# EUROPÄISCHE PATENTANMELDUNG

(11) **EP 4 834 422 A2**
(43) Veröffentlichungstag der Anmeldung: **04.11.2026**
(21) Anmeldenummer: 26204953.9
(22) Anmeldetag: 21.07.2017
(51) Int. Cl.: A61N 2/02

(54) **VORRICHTUNG ZUR BEEINFLUSSUNG BIOLOGISCHER ABLÄUFE IN EINEM LEBENDEN GEWEBE**

(30) Priorität: 24.11.2016 DE 102016122691
(62) Dokumentnummer(n) der früheren Anmeldung(en) nach Art. 76 EPÜ: 17751655.6 / 3 544 675
(71) Anmelder: BEMER Int. AG, 9495 Triesen (LI)
(72) Erfinder: GLEIM, Peter, 9495 Triesen (LI)
(74) Vertreter: Dantz, Dirk

(57) **Zusammenfassung**

Die vorliegenden Erfindung richtet sich auf eine Vorrichtung zur Beeinflussung biologischer Abläufe in einem lebenden Gewebe, insbesondere einem menschlichen Körper, zur Beaufschlagung zumindest eines Teils des Gewebes mit einem pulsierenden magnetischem Feld, mit einer Felderzeugungsvorrichtung (2) zur Erzeugung des pulsierenden magnetischen Feldes und einem Impulsgenerator (1) zur Ansteuerung der Felderzeugungsvorrichtung (2), wobei der Impulsgenerator (1) derart ausgebildet ist, dass das pulsierende Magnetfeld aus einer Folge von Hauptimpulsen (10) besteht deren Impulswiederholungsrate zwischen 0,01 und 1000 Hz liegt, wobei die Hauptimpulse (10) durch eine Mehrzahl von sich überlagernden Unterimpulsen (11) gebildet wird, eine Abfolge von Hauptimpulsen (10) von einer zweiten Abfolge von Hauptimpulsen (10) durch eine Ruhepause (13) unterbrochen ist, wobei der Verlauf der Maximalamplitude der Hauptimpulse (10) zu Beginn der Ruhepause (13) eine Steigung mB von -∞ < mB < -0,1 und/oder zum Ende der Ruhepause (13) eine Steigung mE von ∞ > mE > 0,1 aufweist.

## Beschreibung

Die Erfindung betrifft eine Vorrichtung und ein elektrisches oder elektromagnetisches Signal zur Beeinflussung biologischer Abläufe in einem lebenden Gewebe, insbesondere einem menschlichen Körper, durch Beaufschlagung zumindest eines Teils des Gewebes mit einem pulsierenden elektromagnetischen Feld.

Vorrichtungen, die elektromagnetische Felder erzeugen und die routinemäßig in Kliniken, speziell im Bereich der Orthopädie, zu therapeutischen Zwecken eingesetzt werden, sind seit Beginn der 70iger Jahre bekannt. Die sinusförmigen Magnetfelder, die in semiinvasiven Verfahren mit pulsierenden Magnetfeldern verwendet wurden, wiesen eine Frequenz von 2 bis 20 Hz und magnetische Flussdichten zwischen 1mT und 10 mT auf. An implantierten Elektroden wurde mit Hilfe eines sog. Sekundärelementes eine Wechselspannung zur Erzeugung eines externen Magnetfelds induziert.

Weiterhin war aber auch die nichtinvasive Behandlung ohne Sekundärelement bekannt, bei der im behandelten Körperteil, der sich im Zentrum der Spule zu befinden hatte, nur sehr schwache elektrische Ströme induziert wurden. Auch Geräte für die Ganzkörpertherapie sind seit den 70ger Jahren bekannt, bei dem sich die Feldlinien gleichmäßig im Körper verteilen.

Bei diesen Behandlungsformen dient dabei ein Generator zur Ansteuerung einer Magnetfelderzeugungsvorrichtung, bei dem der Generator die Magnetfelderzeugungsvorrichtung so ansteuert, dass das Magnetfeld aus einer Vielzahl von in ihrem zeitlichem Abstand und Amplitudenverlauf charakteristisch geformten Grundimpulsen bzw. Hauptimpulsen besteht. Die Impulshäufigkeit liegt üblicherweise zwischen 0 und 1000 Hz. Ein derartiger Hauptimpuls kann Sinus-, Trapez- oder auch Sägezahnform (EP 0 594 655 B1 (König Herbert ), EP 0 729 318 B1 (Fischer Gerhard , EP-A-0 377 284 ) haben oder wie in EP 0 995 463 B1 (Kafka Wolf A ) durch einen im Mittel exponentiell ansteigenden sinusförmig modulierten Feldintensitätsverlauf mit magnetischen Flussdichten in Bereichen von nanoTesla bis mehreren milliTesla aufweisen. Weiterhin können sich die Hauptimpulse aus einer Reihe zeitlich aufeinanderfolgender Unterimpulse zusammensetzen, die sich in ihren Amplituden und/oder Anstiegs- bzw. Abfall-Steilheiten, letztlich somit auch in ihrer individuellen Dauer unterscheiden (vgl. EP 0 995 463 B1).

Die magnetischen Felder werden häufig durch ein oder mehrere voneinander auch unabhängig angesteuerten elektrischen Spulen erzeugt ( EP 1 364 679 A2, EP-A 0 266 807, EP-A-0 266 907, DE-A 4 221 739, US-A-5 181 902, WO-A-96/32159, UA-A-4 428 366 , EP 0 995 463 B1). In der heutigen Zeit erfolgt die therapeutische Applikation aus Gründen des operativen Aufwands und der damit verbundenen Risiken üblicherweise nichtinvasiv.

Nach gängiger Vorstellung beruht die Beeinflussung des biologischen Systems auf einem noch unbekannten Zusammenwirken von Energieanteilen der durch die Vorrichtungen generierten magnetischen und elektrischen Feldanteile. Die durch das angelegte elektrische und magnetische Feld ausgelösten physiologischen und biologischen Wechselwirkungen basieren danach energetische Aktivierung der Reaktivität von Molekülstrukturen, die an den naturgegebenen und auf dem Selbsterhalt ausgerichteten Regulationsmechanismen. Die energetische Aktivierung kann direkt, durch magnetische oder/und nach dem Prinzip der Induktion (Maxwellschen Gleichungen) und indirekt, durch elektrische Kraftwirkungen ausgelöst werden. Die Molekülstrukturen können hingegen ionale, atomare und molekulare Form aufweisen.

Beispielsweise beschreibt die EP 0 995 463 B1, dass ein elektromagnetisches Feld gegenüber unbeaufschlagten biologischen Objekten zur einer signifikanten Aktivierung einer Reihe differenzierter physikalisch-physiologisch Prozesse führt. So wurde beispielsweise von
- Der Bildung energiereicher Verbindungen insbesondere von Adenosintriphosphat (ATP) und Bis-2-3-phospoglycerat (BPG) in humanen Erythrozyten beobachtet [Spodaryk K (2001) Red blood metabolism and haemoglobin oxygen affinity: effect of electromagnetic fields on healthy adults. In: Kafka WA (ed) 2nd Int World Congress Bio-Electro-Magnetic-Energy-Regulation. Emphyspace 2: 15-19 ; Kafka WA, Spodaryk K (2003) Effects of extremely weak BEMER 3000 type pulsed electromagnetic fields on red blood metabolism and hemoglobin oxygen affinity. Fizoterapia 11 (3): 24-31 ].
- Der Verbesserung des Funktionszustands der Mikrozirkulation insbesondere hinsichtlich des Durchblutungsverhaltens (besonders auch bei Diabetes-bedingten Durchblutungsstörungen) und der Sauerstoffutilisation [Klopp R (2004) Vitalmikroskopische und reflexionsspektrometrische Untersuchungen zur Wirkung des Gerätesystems "BEMER 3000" auf den Funktionszustand der Mikrozirkulation. Bericht aus dem Institut für Mikrozirkulation, Berl in; Klopp R, Niemer W (2007) Einfluss eines pulsierenden elektromagnetischen Feldes mit vasomotorischer Stimulation auf einen eingeschränkten Funktionszustand der Mikrozirkulation. Komplement. Integr. Med 08/2007: 47-53 ].
- Der Beschleunigung des Ablaufs von Schutzmechanismen insbesondere hinsichtlich eines in Klopp 2004 beschriebenen beschleunigten Ablaufs infektiös ausgelöster, durch komplexes Zusammenspiel von Signal- und Adhäsionsmolekülen getragener Leukozyten-Immunabwehrreaktionen
- Der Protektion gegen chemische Stressfaktoren insbesondere der Reduktion chemisch (durch das Teratogen Cyclophosphamid) induzierter Missbildungen in der Ontogenese von warmblütigen Wirbeltierembryonen (am Model von Hühnereiern) [Jelínek R, Bláha J, Dbalý Jaroslav (2002) The electromagnetic BEMER 3000 signal modifies response to teratogens. In: Kafka WA (ed) 3nd Int. World Congress Bio-Electro-Magnetic Energy-Regulation, Bad-Windsheim, Germany , Emphyspace 3].
- Der verbesserten Heilung von standardmässig erzeugter Wunden [Kafka WA, Preißinger M (2002) Verbesserte Wundheilung durch gekoppelte, BEMER 3000 typisch gepulste, Elektromagnetfeld- und LED-Licht-Therapie am Beispiel vergleichender Untersuchungen an standardisierten Wunden nach Ovariektomie bei Katzen (felidae). In: Edwin Ganster (Hrsg) Österreichische Gesellschaft der Tierärzte (ÖGT) Kleintiertage-Dermatologie 2.-3. März 2002, Salzburg Congress ]
- Anti-oxidativen Regulationen insbesondere hinsichtlich enzymatisch und spektralphotometrisch bestimmter beschleunigter Reduktionsumsatzraten [Spodaryk K (2002) The effect of extremely weak electromagnetic field treatments upon signs and symptoms of delayed onset of muscle soreness: A placebo controlled clinical double blind study. Medicina Sportiva 6: 19-25 ; Klopp R, Niemer W, Pomrenke P, Schulz J (2005) Magnetfeldtherapie: Komplementär-therapeutisch sinnvoll oder Unsinn? Stellungnahme unter Berücksichtigung neuer Forschungsergebnisse mit dem Gerätesystem BEMER 3000, Institut für Mikrozirkulation, Berl in]
- der Leistungsteigerung im Spitzensport [Spodaryk K and Kafka WA (2004) The influence of extremely weak BEMER3000 typed pulsed electromagnetic fields on ratings of perceived exertion at ventilatory threshold. In: Marincek C, Burger H (eds) Rehabilitation Sciences in the New Millennium Challenge for Multidisciplinary Research. 8th Congress of EFRR, Ljubljana. Medimont International Proceedings: 279-283 ]
- von Replikations- und Proliferationsmechanismen insbesondere hinsichtlich einer signifikanten Reduktion des Tumorwachstums in thymusfreien nicht aber in vergleichend untersuchten normalen Mäusen [Rihova B (2004) Die Wirkung der elektromagnetischen Felder des BEMER 3000 auf das Wachstum des experimentellen Mäuse-EL 4T Zellen-Lymphoms, SAMET Kongress, Interlak en; Rihova B, Dbaly J, Kafka WA Exposure to special (BEMER-type) pulsed weak electromagnetic fields does not accelerate the growth of mouse EL4 T cell lymphoma, submissed]
- der Proteinbildung und -Aktivierung insbesondere hinsichtlich differentieller up- und down- Regulation genexprimierter Proteinmengen. Im Rahmen einer Genchip-Analyse konnte gezeigt werden, dass die Anwendung der Vorrichtung EP 0 995 463 B1 auf Stammzellen des Knochenmarks (Knochen und Knorpelzellen) gegenüber nichtbehandelten die Menge der produzierten Proteine unterschiedlich beeinflusst: die Menge (Expression) der produzierten Proteine ist also teils erhöht, teils erniedrigt, teils bleibt sie (interessanterweise unter anderem hinsichtlich der Expression von Onkogenen) unbeeinflusst [Kafka WA, Schütze N, Walther M (2005) Einsatz extrem niederfrequent (BEMER typisch) gepulster schwacher elektromagnetischer Felder im Bereich der Orthopädie (Application of extreme low frequent (BEMER type) pulsed electromagnetic fields in orthopedics). Orthopädische Praxis 41 (1): 22-24 ; Walther M, Meyer F, Kafka WA, Schütze N (2007) Effects of weak, low frequency pulsed electromagnetic fields (BEMER type) on gene expression of human mesenchymal stem cells and chondrocytes: an in vitro study. Electromagnetic Biology and Medicine, Manuscript ID: 257936 ].
- psychovegetativer Prozesse insbesondere der Reduktion der (Zahnarzt-) Angst durch eine der Zahnbehandlung unmittelbar vorrausgehende elektromagnetische lokale Stimulation des Solarplexus [Michels-Wakili S and Kafka WA (2003) BEMER 3000 pulsed low-energy electromagnetic fields reduce dental anxiety: a randomized placebo controlled single-blind study. 10th International Congress on Modern Pain Control 5-8 June 2003 Edinburgh, GB ]
- der Reduktion lumbargisch initiierter Folgereaktionen insbesondere der Reduktion von Bewegungsschmerz, Schlaflosigkeit und Angst [Bernatzky G, Kullich W, Aglas F, Ausserwinkler M, Likar R, Pipam W, H. Schwann H, Kafka WA (2007) Auswirkungen von speziellen, (BEMER-typisch) gepulsten elektro-magnetischen Feldern auf Schlafqualität und chronischen Kreuzschmerz des Stütz- und Bewegungsapparates (low back pain): Eine doppelblinde randomisierte Duo Center Studie (Der Schmerz, in press ].
- der analgetischen Wirkung, insbesondere hinsichtlich der Reduktion polyneuropathischer Schmerzzustände als Folge von oxydativem Stress nach Chemotherapie [Gabrys M (2004) Pulsierende Magnetfeldtherapie bei zytostatisch bedingter Polyneuropathie. Deutsche Zeitschrift für Onkologie 36: 154-156 ].
berichtet.

Über die Wirkungen der elektromagnetischen Beaufschlagung von biologischem Material berichten weiterhin zusammenfassend
- Carpenter DO, Aryapetyan S (1994) Biological effects of electric and magnetic elds: sources and mechanism, vol 1 . Beneficial and harmful effects, Vol 2. Academic Press ;
- Bohn W, Kafka WA (2004) Energie und Gesundheit: BEMER 3000 Bio-ElektroMagnetische-Energie-Regulation nach Prof. Dr. Wolf A. Kafka. Haug Verlag, Stuttgart (Thieme Verlagsgruppe): 1-130 ;
- Kafka WA (2006) The BEMER 3000 Therapy: A new complementary "electro-magnetic drug" effectively supports widespread scattered prophylactic and therapeutic treatments. In: Kochueva E (ed) Achievements in space medicine into health care practice and industry 3rd European praxis matured congress KOPIE-DRUCK sponsored by ESA, DLR & POCKO MOC ];
- Quittan M, Schuhfried O, Wiesinger GF, Fialka-Moser V (2000) Klinische Wirksamkeiten der Magnetfeldtherapie - eine Literaturübersicht. Acta Medica Austriaca 3:61-68 ;
- Matthes Rudiger (2003) Guidance on determining compliances of exposure to pulsed and complex non sinusoidal waveforms below 100 khz with ICNIRP GUIDELINES. The International Commission on Non-Ionizing Radiation Protection ICNIRP Secretariat, Bundesamt für Strahlenschutz, Institut für Strahlenhygiene, Ingolstädter Landstrasse, D-85764 Oberschleissheim, Germany ].

Aufgrund der differenzierten Wirkung auf das Tumorwachstum und die Genexpression, lassen sich die Wirkungen nicht mit einer verbesserten Mikrozirkulation erklären, sondern bestätigen und implizieren die einleitend aufgeführte Annahme, dass die elektromagnetisch induzierte biologische Wirkung auf der Aktivierung ursächlich unterschiedlicher molekularen Mechanismen beruhen. Es wird angenommen, dass die unterschiedlichen Prozesse demzufolge unterschiedliche Energiemengen zu ihrer Aktivierung benötigen. Der Verteilung der Amplituden, der Ausgestaltung der Flankensteilheiten und der Überlagerung der Unterimpulse kommt daher eine entscheidende Bedeutung zu, da mit diesen Parametern die Intensitätsverteilung über der Zeit gekennzeichnet wird. Den zeitlichen Feldintensitätsverteilungen kommt daher eine ähnliche Bedeutung zu, wie der Struktur-Aktivitätsbeziehung von Arzneimittelwirkstoffen in der Pharmazie.

Da derzeit nur schwachenergetische Vorrichtungen zum Einsatz kommen, sind weiterhin auch keine schädlichen Nebenwirkungen zu erwarten. Dies bestätigen Berichte der WHO [Electromagnetic Fields (EMF) ff. http://www.who.int/peh-emf/en/; http://www.who.int/topics/electromagnetic_fields/en/; ] und ein Bericht einer für die Zertifizierung von Medizinprodukten zuständigen deutschen Aufsichtsbehörde [LGA-Bericht 2005], der dokumentiert, dass speziell für die - nach statistischen Erhebungen seit 1998 bis heute abgeschätzt mehrere millionenfach eingesetzte - Vorrichtung EP 0 995 463 B1 keine gesundheitlich negativen Auswirkungen gefunden wurden.

Die EP 0 995 463 B1 beschreibt weiterhin die Beaufschlagung von biologischem Material mit elektrischen oder elektromagnetischen Feldern. Die Beaufschlagung findet hier in zwei Intervallen statt, wobei sich die Frequenz des elektrischen oder elektromagnetischen Feldes im ersten Intervall von der Frequenz des elektrischen oder elektromagnetischen Feldes im zweiten Intervall unterscheiden kann. In einer dargestellten Ausführungsform der EP 0 995 463 B1 sind die Intervalle, in denen das biologischen Material mit elektrischen oder elektromagnetischen Feldern beaufschlagt wird, von einem dritten Intervall unterbrochen, in dem keine Beaufschlagung des biologischen Materials stattfindet., die Amplitude des elektrischen oder elektromagnetischen Feldes dementsprechend gleich 0 ist.

In einer in der EP 2 050 481 A1 beschriebenen Weiterentwicklung wurde der Intensitätsverlauf über die Zeit dahingehen angepasst, dass die Impulse feiner an die Anforderungen der Therapie angepasst werden. Die optimale Form und Abfolge der Unterimpulse ist individuell sehr verschieden. Sie hängt ab von der Art des vom Feld beaufschlagten Gewebes, von dem erwünschten Heilungserfolg und vom jeweiligen Individuum. Eine entscheidende Bedeutung bei der Stimulation der Austauschprozesse im Körpergewebe hat vermutlich der hohe, durch die Vielzahl der überlagerten Unterimpulse bedingte Anteil der an- bzw. absteigenden Flankenabschnitte. Weiterhin wird in der EP 2 050 481 A1 beschrieben, dass zwischen zwei Intervalle mit einer Beaufschlagung des biologischen Gewebes mit einem elektromagnetischen oder elektrischen Feld durch eine Pause unterbrochen sein kann, in der keine Beaufschlagung stattfindet..

Infolge der noch nicht vollständig verstandenen Prozesse in den mit dem elektromagnetischen Feld beaufschlagten biologischen Geweben ist es allerdings bisher noch nicht gelungen eine optimierte Pulsfolge zu entwickeln. Alle bisher bekannten Vorrichtungen zur Behandlung des menschlichen Körpers führen daher nicht immer zu der erwünschten beschleunigenden Wirkung des Heilungsvorgangs. Es ist insbesondere problematisch, dass bei bisherigen Vorrichtungen zur Erzielung eines deutlich beschleunigten Heilerfolges die Anwendung nicht derart effektiv arbeiten, dass eine Behandlung mit häufigen Wiederholungen durchgeführt werden muss. Dies führt zu einer erhöhten Belastung der Patienten und im Ergebnis zu deutlich höheren Behandlungskosten.

### Aufgabe

Aufgabe der Erfindung ist es, gegenüber dem Stand der Technik eine verbesserte Vorrichtung und ein verbessertes elektrisches oder elektromagnetisches Signal zu schaffen, mit denen eine schnellere und in ihrer physiologischen Wirkung breitere Beeinflussung, insbesondere Anregung, biologischer Abläufe ermöglicht wird, indem ein breites Band an elektromagnetisch aktivierbaren Molekularstrukturen angesprochen wird und somit eine breitere physiologische Wirkungsbreite gewährleistet wird. Die Erfindung ist damit auf eine möglichst breit wirksame energetische Unterstützung der komplex vernetzten molekularen Regulationsprozesse ausgerichtet. Das einhergehende Therapiekonzept ist somit präventiv und auf die Regeneration, Erhaltung und das Wohlbefinden ausgerichtet.

### Problemlösung

Diese Aufgabe wird erfindungsgemäß durch eine Vorrichtung gemäß Anspruch 1 gelöst, die einen Impulsgenerator und eine Felderzeugungsvorrichtung zur Erzeugung eines pulsierenden elektromagnetischen Feldes umfasst. Der Impulsgenerator dient zur Ansteuerung der Felderzeugungsvorrichtung, wobei der Impulsgenerator die Felderzeugungsvorrichtung über geeignete Strom-Spannungsabläufe so ansteuert, dass das pulsierende elektrische oder elektromagnetische Feld aus einer Vielzahl von, hinsichtlich ihres zeitlichen Amplitudenverlaufs charakteristisch geformten Hauptimpulsen besteht, deren Frequenz zwischen 1 und 1000 Hz liegt. Ein derartiger Hauptimpuls kann sich dabei aus einer Überlagerung eines nach einer Potenzfunktion an- oder absteigenden Grundimpulses mit einer Reihe von aufgesetzten Pulsen von jeweils kürzerer Zeitdauer und unterschiedlicher Form und zeitlicher Abfolge aufbauen.

Der zeitliche Amplitudenverlauf eines derartigen Hauptimpulses könnte etwa der folgender Funktion entsprechen: $y \left(x\right) = \frac{{x}^{a} ∗ k ∗ {e}^{sin {x}^{b}}}{c} + d$ darin bedeuten:
y(x) = Magnetfeld-Amplitude innerhalb eines Hauptimpulses als Funktion von x;
x = den Zeitverlauf; wobei x für jeden Hauptimpuls wieder von neuem mit demselben Anfangswert beginnt
a = ein Parameter zur Einstellung des zeitlichen Amplitudenverlaufs jedes Hauptimpulses (Hüllkurve);
b = die Anzahl der Unterimpulse
c = ein Faktor zur Einstellung der Amplitude
d = ein Offsetwert;

Die Hauptimpulse sind erfindungsgemäß in Impulsgruppen zusammengefasst, wobei zwischen den Hauptimpulsen eine Ruhezeit vorgesehen ist. Zwischen zwei aufeinander folgenden Impulsgruppen sind zudem Ruhephasen vorgesehen, wobei die Ruhephasen (13) von der Ruhezeit verschieden sind. Weiterhin liegt das Tastverhältnis zwischen Ruhezeit und Hauptimpuls (10) zwischen 3:1 bis 1:3.

Die Ruhephasen haben Erfindungsgemäß eine Dauer von einer Vielzahl von Hauptimpulsen, die mindestens größer als 10 Hauptimpulse ist, wobei ein Frequenzwechsel der Frequenz des Hauptimpulses einer Impulsgruppe vor der Ruhephase zu der Frequenz einer Impulsgruppe nach der Ruhephase während der Ruhephase erfolgt.

Die Vorrichtung ist hierbei derart gestaltet, dass zwischen zwei aufeinander folgenden Impulsgruppen der Hauptimpulse eine Ruhepause vorgesehen ist. Es hat sich gezeigt, dass vermutlich aufgrund der Relaxationszeit der Austauschprozesse eine solche Ruhepause einen positiven Effekt auf die molekularen Mechanismen innerhalb des behandelten Körpergewebes hat und somit einen besseren Therapieerfolg zeigt. Die molekularen Prozesse können während der Ruhepause ihre normale Funktion weitestgehend wieder aufnehmen. Diese Ruhepause ist erfindungsgemäß derart gestaltet, dass der Verlauf der Maximalamplitude der Hauptimpulse zu Beginn der Ruhepause eine Steigung m im Bereich von -∞ < m < 0.1 aufweist und/oder zum Ende der Ruhepause eine Steigung m von -∞ > m > 0.1. Die Amplituden der Hauptimpulse fallen zu Beginn der Pause sehr stark, um die biologische Wirkung der elektromagnetischen Impulse schnell zu reduzieren und die Relaxation der molekularen Prozesse schnell zu erreichen. Damit die Aktivierung der molekularen Prozesse am Ende der Ruhepause wieder schnell einsetzt, steigt die Maximalamplitude der Hauptimpulse am Ende der Ruhepause steil an.

Die Absenkung der Maximalamplitude der Hauptimpulse beträgt in einer weiteren Ausführung der Erfindung im Bereich der Ruhepause mindestens 40 %, bevorzugt 25 % und besonders bevorzugt 15 % der Maximalamplitude der Impulsgruppe vor Beginn und/oder nach Ende der Ruhepause. Die Aktivierung der molekularen Prozesse im behandelten Körpergewebe wird also während der Ruhepause nicht vollständig ausgeschaltet, sondern mit signifikant niedrigerer Intensität fortgesetzt. Die Stimulierung z.B. der Vasomotorik kleiner und kleinster Blutgefäße wird damit beibehalten und ermöglicht so einen günstigeren Blutstrom im behandelten Körpergewebe.

In einem weiteren Aspekt der Erfindung beträgt die Dauer der Ruhepause mindestens 0,1 s, bevorzugt 1 s und besonders bevorzugt 3 s. Es hat sich gezeigt, dass die molekularen Austauschprozesse während dieser Zeitspanne vermutlich ausreichend relaxieren und ihre normale Funktion weitestgehend wieder aufnehmen können. Während dieser Ruhepause beträgt wie oben dargelegt die Absenkung der Maximalamplitude der Hauptimpulse mindestens 40 %, bevorzugt 25 % und besonders bevorzugt 15 % der Maximalamplitude der Impulsgruppe vor Beginn und/oder nach Ende der Ruhepause.

In einer weiteren Ausführung der Erfindung verläuft die Maximalamplitude der Hauptimpulse im Bereich der Ruhepause konstant. Während der Ruhepause werden Schwankungen der Maximalamplituden der Hauptimpulse vermieden, um die Aktivierung molekularer Mechanismen auf niedrigerem Niveau zu gestalten.

Es hat sich gezeigt, dass ein zu schnelles Herunterfahren der Maximalamplitude der Hauptimpulse zu Beginn der Ruhepause den Therapieverlauf stört, weil vermutlich die unterschiedlichen molekularen Prozesse ebenfalls unterschiedlich relaxieren. Daher beträgt in einer weiteren Ausgestaltung der Erfindung zu Beginn der Ruhepause die Abschwelldauer der Maximalamplitude der Hauptimpulse zwischen 1/4 und 1/3 der Gesamtdauer der Ruhepause. Damit wird gewährleistet, dass während der Ruhepause alle molekularen Mechanismen durch das elektromagnetische Signal weitestgehend deaktiviert sind.

Alternativ oder zusätzlich beträgt in einer weiteren Ausführungsform der Erfindung die Anstiegsdauer der Maximalamplitude der Hauptimpulse zum Ende der Ruhepause zwischen 1/4 und 3/4 der Gesamtdauer der Ruhepause. Dieser Prozess bewirkt eine gleichmäßige und für den Patienten schonendere Anregung der molekularen Mechanismen im behandelten Gewebe.

In einer weiteren Gestaltung der Erfindung beträgt das Verhältnis der mittleren Steigung des Verlaufs der Maximalamplitude des Hauptimpulses zu Beginn der Ruhepause mB und der mittleren Steigung des Verlaufs der Maximalamplitude des Hauptimpulses am Ende der Ruhepause mE erfindungsgemäß zwischen -1 > mB/mE > -10. d.h. am Ende der Ruhepause steigt die Maximalamplitude des Hauptimpulses gleich oder um die genannten Faktoren geringer, als der Hauptimpuls zu Beginn der Ruhepause abschwillt. Erfahrungsgemäß sollte die Anregung am Ende der Ruhepause geringer ansteigen, als die Anregung zu Beginn der Ruhepause abschwillt. So wird gewährleistet, dass einerseits alle molekularen Prozesse gleichmäßig zu Beginn der Ruhepause nicht mehr angeregt werden. Auf der anderen Seite erfolgt die Anregung am Ende der Ruhepause für den Patienten schonender und zeigt damit bessere Therapieerfolge.

Während der Ruhepause erfolgt in einer besonderen Ausgestaltung der Erfindung ein Frequenzwechsel der Frequenz des Hauptimpulses einer Impulsgruppe. Erfindungsgemäß weist der Wert der Frequenz des Hauptimpulses einer Impulsgruppe vor der Ruhepause einen anderen Wert auf als der Wert der Frequenz des Hauptimpulses einer Impulsgruppe nach der Ruhepause. Die Intensitätsverteilung der Impulse über die Zeit ändert sich also nach der Ruhepause. Die elektromagnetisch induzierte Aktivierung wirkt nach der Ruhepause auf andere molekulare Mechanismen als vor der Ruhepause. Durch diesen erfindungsgemäßen Frequenzwechsel wird also eine breitere Anregung, Aktivierung der Prozesse und ein besserer Therapieerfolg erzielt.

Im Folgenden werden die Begriffe "Einzelimpuls" und "Ruhephase" synonym zu den bereits eingeführten Begriffen "Hauptimpuls" und "Ruhepause" verwendet.

Anhand der Zeichnung wird die Erfindung näher erläutert. Es zeigt:
- Fig. 1: Erfindungsgemäße Vorrichtung
- Fig. 2: a) Unterimpulse eines Einzelimpulses
b) Aus Unterimpulsen zusammengesetzter Einzelimpuls
c) Vereinfachte Darstellung eines Einzelimpulses
- Fig. 3: Einzelimpulse unterbrochen von einer Ruhephase mit gleichbleibender Amplitude während der Ruhephase
- Fig. 4: Einzelimpulse unterbrochen von einer Ruhephase mit unterschiedlich steilen Amplitudenverläufen zu Beginn und Ende der Ruhephase
- Fig. 5: Einzelimpulse unterbrochen von einer Ruhephase mit gleichbleibender Amplitude während der Ruhephase und unterschiedlich steilen Amplitudenverläufen zu Beginn und Ende der Ruhephase
- Fig. 6: Einzelimpulse unterbrochen von einer Ruhephase mit gleichbleibender Amplitude während der Ruhephase unterschiedlich steilen Amplitudenverläufen zu Beginn und Ende der Ruhephase

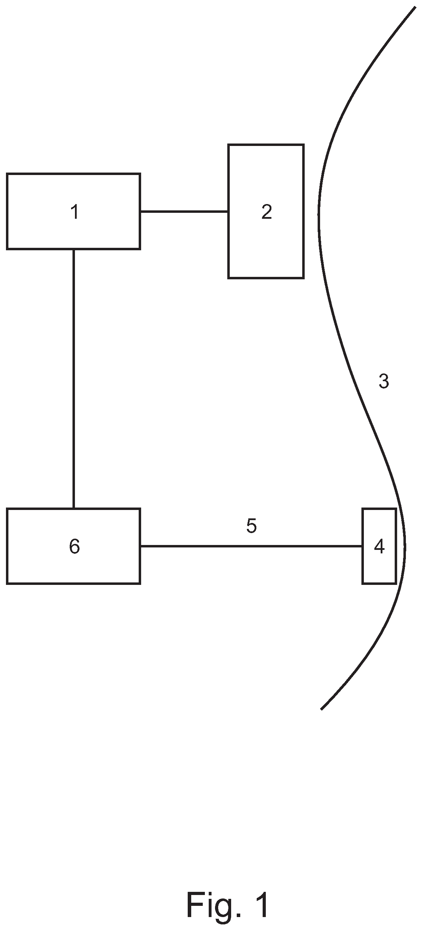

Im Einzelnen zeigt Fig. 1 eine erfindungsgemäße Vorrichtung, die zumindest einem Impulsgenerator 1 umfasst, der in der Spule 2 ein pulsierendes magnetisches Feld erzeugt. Das Feld wechselwirkt mit dem lebenden Gewebe 3, insbesondere einem Körper eines zu behandelnden Patienten. Weiterhin umfasst die erfindungsgemäße Vorrichtung einen Sensor, mit dem Körperparameter wie beispielsweise die Temperatur, der Blutdruck, die Pulsfrequenz oder der Sauerstoffgehalt des Blutes erfasst werden können. Über die Rückkopplungsleitung 5 werden die erfassten Körperparameter an ein Steuergerät 6 gesendet. Über die Parameter und entsprechende Algorithmen können die erfassten Körperparameter ausgewertet und das pulsierende Magnetfeld im Generator 1 optimiert werden. Es ist möglich mehrere Parameter zeitgleich zu erfassen und zu optimieren, um ein effektives pulsierende Magnetfeld einzustellen. In Abhängigkeit von diesen Wirkungen kann das Steuergerät 6 jeweils die optimalen Werte für die Parameter a bis d und k auch automatisch festlegen.

Weiterhin kann über den Sensor 4 die Wirkung des pulsierenden Magnetfeldes auf den zu behandelnden Körper erfasst werden und in Abhängigkeit verschiedener Parameter des pulsierenden Magnetfeldes gesetzt werden. Solche Parameter sind beispielsweise die Frequenz von Einzel, Haupt- Neben- und/oder Unterimpulsen oder die Amplitude dieser Impulse. Aus den Unterschieden, insbesondere in der spektralen Zusammensetzung zwischen der von der Felderzeugung erzeugten Feldenergie und dem vom Sensor erfassten Magnetfeld, ermittelt das Steuergerät den auf den behandelten Körper übertragenen Anteil. Über das Steuergerät können die Parameter des pulsierenden Magnetfeldes (a bis d und k) angepasst und hinsichtlich der Behandlungswirkung optimiert werden.

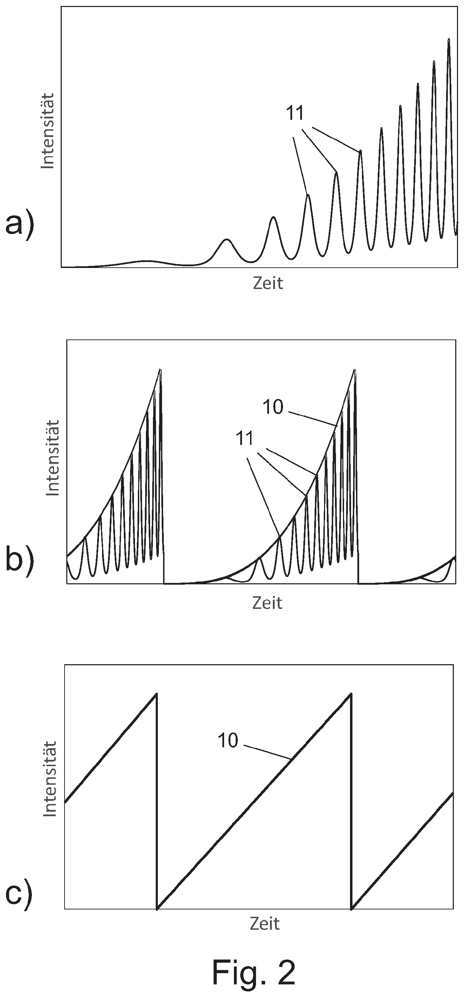

Mit Hilfe der erfindungsgemäßen Vorrichtung und des erfindungsgemäßen Verfahrens wird nun ein pulsierende Magnetfeld erzeugt. Das Magnetfeld weist eine Abfolge von Hauptimpulsen 11 auf, deren Verlauf in Bezug auf Amplitude und Zeit prinzipiell den in Fig. 2 b dargestellten Verlauf entspricht. In Fig. 2 c ist eine vereinfachte Form den Amplitudenverlauf dargestellt. Die Form der Amplitudenverläufe ist abhängig von den Parametern a bis d. Jeder Hauptimpuls 11 ist hierbei zusammengesetzt aus einer Abfolge von Unterimpulsen 12. Die Maximalintensitäten dieser Unterimpulse 12 steigen im Verlauf eines Hauptimpulses 11 an.

Die Hauptimpulse 11, aus denen sich das gepulste Magnetfeld zusammensetzt beginnen zu einem Zeitpunkt t1 und erreichen ihren mittleren Mindest- oder Maximalwert. Die mittlere Amplitude des Hauptimpulses 11, bzw. die hierin periodisch modulierten Amplituden steigen bzw. fallen im Mittel im Ablauf jedes eines Hauptimpulses 11. Zu- bzw. Abnahme erfolgen gemäß einer Exponentialfunktion. Es sind aber auch andere Funktionen denkbar die den mittleren Anstieg (Abfall) der Amplitude eines Hauptimpulses 11 innerhalb der Zeit beschreiben. Die optimale Form der Abfolge der Unterimpulse 12 ist individuell sehr verschieden. Sie hängt ab von der Art des vom Feld beaufschlagten Gewebes, von dem gewünschten Heilerfolg und von dem jeweiligen Individuum.

Zwischen den Einzelimpulsen 10 kann sich eine kurze "Ruhezeit" bestimmter Länge befinden, die vermutlich aufgrund der Relaxationszeit der Austauschprozesse erforderlich ist und erfahrungsgemäß zu einer besseren Anregung des lebenden Körpergewebes führt. Das Tastverhältnis zwischen Ruhezeit und aktiver Impulszeit kann zwischen 3:1 bis 1:3 schwanken, vorzugsweise beträgt es in etwa 1:1. Sie liegen beispielsweise in der Grössenordnung 0 bis 200 ms. Das Tastverhältnis zwischen Ruhezeit (Zeitpunkte ta bis tb) und Impulswiederholdauer T liegt vorzugsweise zwischen 0% und 300%. In den manchen Anwendungsfällen ist die Ruhepause jedoch entbehrlich.

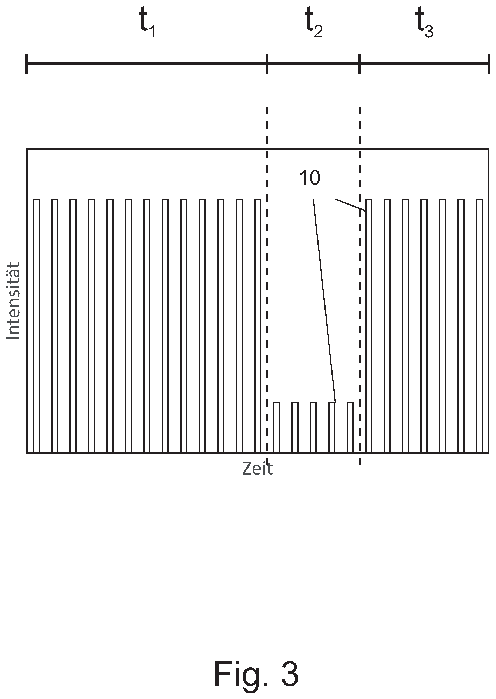

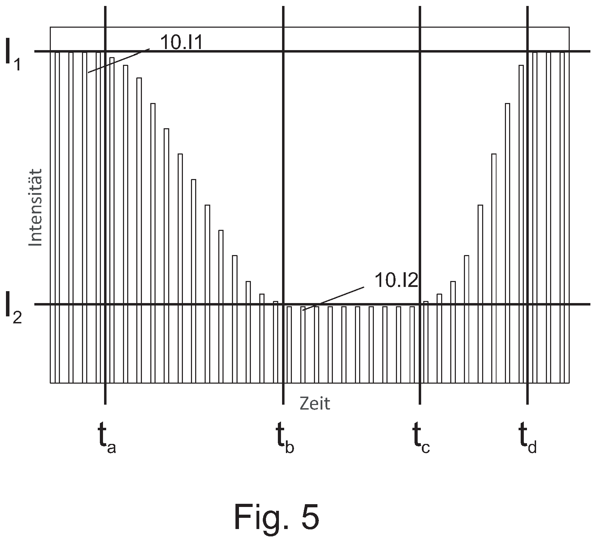

Weiterhin kann die Abfolge einer Mehrzahl der Einzelimpulse 10 durch Ruhephasen 13 getrennt sein vgl. Fig. 3. Diese Ruhephasen 13 haben eine Dauer (tB in Fig. 5) von einer Vielzahl von Einzelimpulsen 10, die mindestens größer als 10 Einzelimpulse ist. Die Dauer der Ruhephase13 tB liegt bevorzugt bei über 0,5 s, besonders bevorzugt bei über 2 s. Während dieser Ruhephasen 13 wird die durchschnittliche Maximalamplitude I der Einzelimpulse 10 auf kleiner gleich 30% der Maximalamplitude der Einzelimpulse in tA abgesenkt. Eine Absenkung auf 0 ist ebenfalls möglich. In dieser Ruhephase 13 wird dem beaufschlagten Gewebe die Gelegenheit zur Regeneration und Relaxation gegeben.

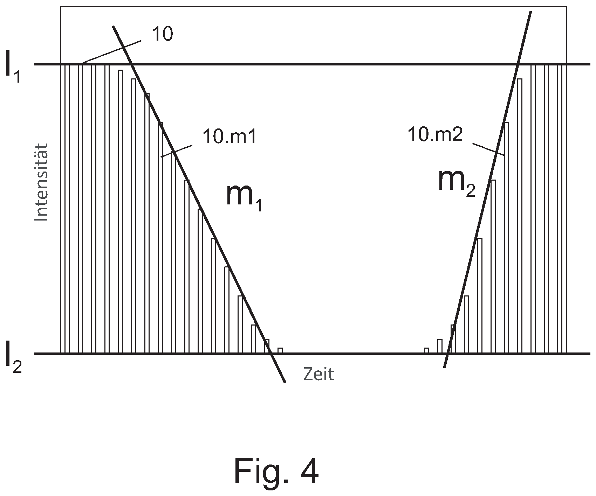

Fig. 4 zeigt eine erfindungsgemäßes Ausführungsbeispiel für eine Ausgestaltung der Ruhephase 13. Zunächst liegt eine Abfolge von Einzelimpulsen 10 vor, die eine konstante Maximalamplitude I aufweisen. Zum Zeitpunkt t₁ beginnt die Maximalamplitude I der Einzelimpulse 10 kontinuierlich abzunehmen bis zu einem Zeitpunkt t₂ ein Minimum der Maximalamplitude I der Einzelimpulse 10 erreicht ist. In dem Zeitintervall zwischen t₁ und t₂ weist die mittlere Steigung mB der Maximalamplitude I einen Wert von mB = -0,12 auf. Es folgt ein Zeitintervall zwischen t₂ und t₃, in dem die abgesenkte Maximalamplitude I der Einzelimpulse 10 gleich 0 ist. Das Zeitintervall zwischen t₂ und t₃ ist 0,12 s lang. Zwischen t₃ und t₄ folgt dann eine Phase, in der die Maximalamplitude I wieder ansteigt. Die mittlere Steigung mE beträgt hier mE = 0,12. Nach t₄ ist die Maximalamplitude I der Einzelimpulse 10 wieder auf dem gleichen Wert wie vor t₁.

In einer ähnlichen nicht bildlich dargestellten Ausführung der Erfindung steigt die Maximalamplitude I der Einzelimpulse 10 nach t₄ auf einen um 20% höheren Wert als die Maximalamplitude I der Einzelimpulse 10 vor t₁. Während des Intervalls zwischen t₂ und t₃ ist die Maximalamplitude I der Einzelimpulse 10 konstant bei 5% der Maximalamplitude I der Einzelimpulse 10 vor t₁.

In Fig. 5 ist ein weiteres Ausführungsbeispiel der Erfindung dargestellt. Nach einer ersten Abfolge von Einzelimpulsen 10 mit konstanter Maximalamplitude I beginnt ab t₁ die Ruhephase 13. Sie weist insgesamt drei Bereiche auf, einen ersten von t₁ bis t₂ auf, in dem die Maximalamplitude I abgesenkt wird, einen zweiten Bereich von t₂ bis t₃, in dem die Maximalamplitude I der Einzelimpulse 10 konstant bei 30% der Maximalamplitude I der ersten Abfolge der Einzelimpulse 10 liegt und einen dritten Bereich zwischen t₃ und t₄, in dem die Maximalamplitude I der Einzelimpulse 10 wieder ansteigt. Nach t₄ weisen die Maximalamplituden I der Einzelimpulse 10 die gleiche Werte auf, wie vor t₁. Während die mittlere Steigung m während des Absenkens der Maximalamplitude I während der ersten Phase der Ruhephase 13 bei mB = -2 liegt, weist sie in der dritten Phase der Ruhephase 13 einen Wert von mE = 3 auf. Das Verhältnis der mittleren Steigung der ersten Phase der Ruhephase 13 zur mittleren Steigung der dritten Phase der Ruhephase 13 liegt somit bei mB/mE = -2/3. Die zweite Phase der Ruhephase 13 ist 1,2 s lang.

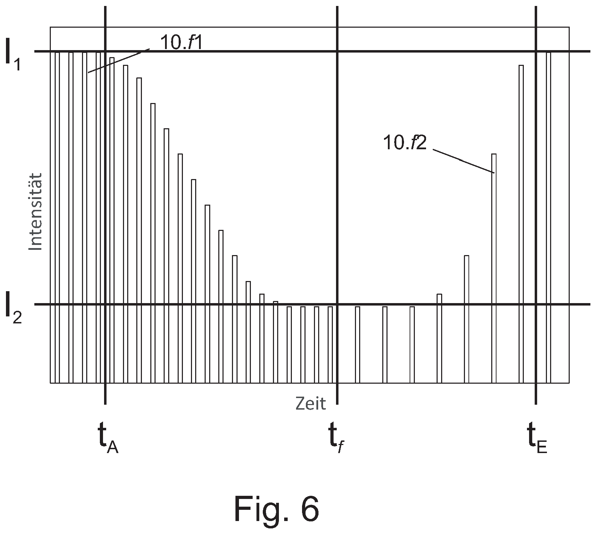

Bei einer weiteren vorteilhaften Ausgestaltung der Erfindung wird die Maximalamplitude I der Einzelimpulse 10 für eine längere zweite Phase auf ein konstantes Niveau von 20% der Maximalamplitude I der Einzelimpulse 10 vor t₁ abgesenkt (Fig. 6). Die zweite Phase beträgt hier 3,6 s. Es zeigt sich aber auch hier, dass die Ausgestaltung der ersten Phase und der dritten Phase der Ruhephase 13 einen entscheidenden Einfluss auf die Stimulation der physiologischen Austauschprozesse haben. Die mittlere Steigung mB der Maximalamplitude I der Einzelimpulse 10 in der ersten Phase der Ruhephase 13 von t₁ bis t₂ beträgt hier mB = -1, während die mittlere Steigung mE der Maximalamplitude I der Einzelimpulse 10 in der zweiten Phase der Ruhephase 13 von t₂ bis t₃ mE = 0,8 beträgt. Während der zweiten Phase der Ruhephase 13 zwischen t₂ und t₃ wurde zum Zeitpunkt t*_{f}* die Frequenz von *f* = 30 Hz auf *f* = 10 Hz gesenkt.

Neben anderen Effekten führt die individuelle Ausgestaltung der Ruhephase 13 zu einer Stimulation der physiologischen Austauschprozesse und trägt damit entscheidend zur Beschleunigung der angesprochenen Regulations- und Heilungsvorgänge bei. Wichtig ist dabei insbesondere, dass durch die Ausgestaltung der Einleitung der Ruhephase und des Wiederanschwellen der Impulse die Relaxation der durch elektromagnetische Beaufschlagung bereits stimulierten Prozesse im Körpergewebe entscheidenden Einfluss auf den Gesamteffekt hat. Diese Ruhephase und ihre individuell abgestimmte Ausgestaltung bilden hier den wesentlichen Unterschied zu der EP 0 995 463 B1 und der EP 2 050 481 A1.

Nicht nur die optimale Form und Abfolge der Einzelimpulse 10 hängt ab von der Art des vom Feld beaufschlagten Gewebes, von dem erwünschten Heilungserfolg und vom jeweiligen Individuum und ist daher individuell sehr verschieden, sondern auch die Art und Weise, wie das Gewebe in die Relaxationsphase geführt wird. Es hat sich zudem gezeigt, dass die molekularen Austauschprozesse während der Ruhephase vermutlich ausreichend relaxieren und ihre normale Funktion weitestgehend wieder aufnehmen können.

Wenn mit Hilfe von Sensoren bestimmte Parameter des lebenden Gewebes, insbesondere des menschlichen Körpers, erfasst werden, lässt sich neben dem Verlauf jedes resultierenden Einzelimpulses 10 derart an die tatsächlichen Verhältnisse anpassen, dass eine optimale Stimulation erreicht wird, auch die Ruhephasen 13 und ihre Ausgestaltung haben erhebliche Einfluss hierauf sind individuell anzupassen. Dazu werden in Abhängigkeit von den erfassten Gewebeparametern die Höhe der Maximalamplitude der Einzelimpulse 10 während der Ruhephase 13, die mittlere Steigung der Maximalamplitude zu Beginn und zum Ende der Ruhephase 13 und ihre Dauer derart eingestellt, dass Beaufschlagung und Relaxation in einem für dieses Gewebe optimierten Verhältnis stehen.

Eine weitere Optimierung der Wirkung der vorliegenden Vorrichtung auf den Organismus kann durch eine Rückkopplung erzielt werden. Zu diesem Zweck werden Sensoren verwendet, die um Umfeld der Beaufschlagung einen oder mehrere Körperparameter messen, um die Anregung des Organismus durch die elektromagnetischen Pulse zu erfassen. Mit den Sensoren lassen sich Gewebeparameter wie beispielsweise Blutdruck, Temperatur, Puls, ph-Wert oder Atemvolumen messen und im Sinne einer adaptiven Anpassung der Stimulation an die Sensibilität des zu stimulierenden Gewebes zur Optimierung der Parameter der Vorrichtung zur Erzeugung elektromagnetischer Felder verwenden. Insbesondere ließen sich die Anpassungen dynamisch während der Behandlung vornehmen, um so kurzfristigen Änderungen des Körperzustandes Rechnung zu tragen und den Behandlungserfolg weiter zu optimieren. Dies erfolgt über eine Rückkopplungsschleife durch die die durch die Anregung selbst verursachten Sensibilitätsänderungen im beaufschlagten Gewebe ausgeglichen werden können.

### BEZUGSZEICHENLISTE

- 1: Impulsgenerator
- 2: Felderzeugungsvorrichtung
- 3: lebendes Gewebe
- 4: Sensor
- 5: Rückkopplungsleitung
- 6: Steuergerät
- 10: Hauptimpuls
- 11: Unterimpuls
- 10.m1, 10.m2: Hauptimpulse im Bereich der an- bzw. abschwellenden Amplitude
- 10.I1, 10.I2: Hauptimpulse vor/nach bzw. während der Pause
- 10.*f*1, 10.*f*2: Hauptimpuls vor bzw. nach dem Frequenzwechsel
- 12, 12.1, 12.2: Unterimpuls
- 13, 13.1, 13.2: Nebenimpuls
- t, t₁, t₂, t₃,: Zeitpunkte
- t_{A,} t_{B,} t_{C}: Zeitintervalle
- I, I₁, I₂: Amplitude
- *f*, *f*₁, *f*₂: Frequenz des Hauptimpulses
- m, m₁, m₂: Steigung des Maximalamplitudenverlaufs im zu Beginn bzw. Ende der Ruhepause

## Patentansprüche

1. Vorrichtung zur Beeinflussung biologischer Abläufe in einem lebenden Gewebe, insbesondere einem menschlichen Körper, zur Beaufschlagung zumindest eines Teils des Gewebes mit einem pulsierenden magnetischem Feld, mit einer Felderzeugungsvorrichtung (2) zur Erzeugung des pulsierenden magnetischen Feldes und einem Impulsgenerator (1) zur Ansteuerung der Felderzeugungsvorrichtung (2), wobei der Impulsgenerator (1) derart ausgebildet ist, dass das pulsierende Magnetfeld aus einer Folge von Hauptimpulsen (10) besteht deren Impulswiederholungsrate zwischen 0,01 und 1000 Hz liegt, wobei die Hauptimpulse (10) durch eine Mehrzahl von sich überlagernden Unterimpulsen (11) gebildet wird, so dass der Amplitudenverlauf eines Hauptimpulses die folgende Funktion aufweist: $y \left(x\right) = \frac{{x}^{a} ∗ k ∗ {e}^{sin {x}^{b}}}{c} + d$ darin bedeuten:
y(x) = Magnetfeld-Amplitude innerhalb eines Hauptimpulses als Funktion von x;
x = den Zeitverlauf; wobei x für jeden Hauptimpuls (10) wieder von neuem mit demselben Anfangswert beginnt
a = ein Parameter zur Einstellung des zeitlichen Amplitudenverlaufs jedes Hauptimpulses (10);
b = die Anzahl der Unterimpulse (11)
c = ein Faktor zur Einstellung der Amplitude
d = ein Offsetwert;
k = ein Faktor zur Einstellung der Amplitude der Unterimpulse (11),
mit a, b, c ≠0,
wobei die Hauptimpulse (10) in Impulsgruppen zusammengefasst sind,
wobei zwischen den Hauptimpulsen (10) eine Ruhezeit vorgesehen ist,
wobei zwischen zwei aufeinander folgenden Impulsgruppen Ruhephasen (13) vorgesehen sind,
wobei die Ruhephasen (13) von der Ruhezeit verschieden sind,
wobei das Tastverhältnis zwischen Ruhezeit und Hauptimpuls (10) zwischen 3:1 bis 1:3 liegt, und
wobei die Ruhephasen (13) eine Dauer von einer Vielzahl von Hauptimpulsen (10) haben, die mindestens größer als 10 Hauptimpulse ist,
**dadurch gekennzeichnet, dass**
die Ruhephasen (13) eine Dauer von einer Vielzahl von Hauptimpulsen (10) haben, die mindestens größer als 10 Hauptimpulse ist,
wobei ein Frequenzwechsel der Frequenz des Hauptimpulses (10) einer Impulsgruppe vor der Ruhephase (13) zu der Frequenz einer Impulsgruppe nach der Ruhephase (13) während der Ruhephase (13) erfolgt.

2. Vorrichtung nach Anspruch 1
**dadurch gekennzeichnet, dass**
die Absenkung der Maximalamplitude der Hauptimpulse (10) im Bereich der Ruhephase (13) auf mindestens 40% der Maximalamplitude der Impulsgruppe vor Beginn und/oder nach Ende der Ruhephase (13), bevorzugt 25% der Maximalamplitude der Impulsgruppe vor Beginn und/oder nach Ende der Ruhephase (13), besonders bevorzugt 15% der Maximalamplitude der Impulsgruppe vor Beginn und/oder nach Ende der Ruhephase (13) beträgt.

3. Vorrichtung nach Anspruch 1 oder 2
**dadurch gekennzeichnet, dass**
die Ruhephase mit einer Absenkung der Maximalamplitude der Hauptimpulse (10) im Bereich der Ruhephase (13) auf mindestens 40% der Maximalamplitude der Impulsgruppe vor Beginn und/oder nach Ende der Ruhephase (13), bevorzugt 25% der Maximalamplitude der Impulsgruppe vor Beginn und/oder nach Ende der Ruhephase (13), besonders bevorzugt 15% der Maximalamplitude der Impulsgruppe vor Beginn und/oder nach Ende der Ruhephase (13) mindestens 0,1s, bevorzugt 1s und besonders bevorzugt 3s beträgt.

4. Vorrichtung nach einem der Ansprüche 1 bis 3
**dadurch gekennzeichnet, dass**
im Bereich der Ruhephase (13) der Verlauf der Maximalamplitude der Hauptimpulse (10) konstant bleibt.

5. Vorrichtung nach Anspruch einem oder mehreren der Ansprüche 1 bis 4
**dadurch gekennzeichnet, dass**
die Abschwelldauer der Maximalamplitude der Hauptimpulse (10) zu Beginn der Ruhephase (13) zwischen 1/3 und ¼ der Gesamtdauer der Ruhephase (13) liegt.

6. Vorrichtung nach einem oder mehreren der Ansprüche 1 bis 5
**dadurch gekennzeichnet, dass**
die Anstiegsdauer der Maximalamplitude der Hauptimpulse (10) zum Ende der Ruhephase (13) zwischen ¾ und ¼ der Gesamtdauer der Ruhephase (13) liegt.

7. Vorrichtung nach einem oder mehreren der Ansprüche 1 bis 4
**dadurch gekennzeichnet, dass**
ein Frequenzwechsel der Frequenz des Hauptimpulses (10) einer Impulsgruppe vor der Ruhephase (13) zu der Frequenz einer Impulsgruppe nach der Ruhephase (13) während der Ruhephase (13) erfolgt.
